(19) Europäisches Patentamt
European Patent Office
Office européen des brevets

(11) Veröffentlichungsnummer: **0 433 805 A1**

(12) # EUROPÄISCHE PATENTANMELDUNG

(21) Anmeldenummer: **90123604.2**

(22) Anmeldetag: **08.12.90**

(51) Int. Cl.[5]: **C07D 207/34**, C07D 401/12, C07D 405/12, C07D 409/12, A01N 43/36

(30) Priorität: **21.12.89 DE 3942225**

(43) Veröffentlichungstag der Anmeldung: **26.06.91 Patentblatt 91/26**

(84) Benannte Vertragsstaaten: **BE CH DE FR GB IT LI NL**

(71) Anmelder: **BAYER AG**
**W-5090 Leverkusen 1 Bayerwerk(DE)**

(72) Erfinder: **Wollweber, Detlef, Dr.**
**Paul-Ehrlich-Strasse 17**
**W-5600 Wuppertal 1(DE)**
Erfinder: **Dutzmann, Stefan**
**Leinenweberweg 33**
**W-4000 Düsseldorf 13(DE)**
Erfinder: **Brandes, Wilhelm, Dr.**
**Eichendorffstrasse 3**
**W-5653 Leichlingen 1(DE)**
Erfinder: **Hänssler, Gerd, Dr.**
**Am Arenzberg 58a**
**W-5090 Leverkusen 3(DE)**

(54) **N-Substituierte 3-Cyano-4-phenyl-pyrrole.**

(57) Neue Verbindungen der Formel (I),

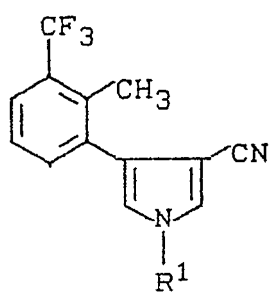

(I)

in welcher
die Substituenten, die in der Beschreibung gegebene Bedeutung haben und ihre Verwendung zur Bekämpfung von Schädlingen. Die Stoffe sind durch die Formel (I) allgemein definiert, sie können nach Analogieverfahren hergestellt werden, z.B. aus einem geeigneten Pyrrol mit Formaldehyd und Aminen oder aus geeigneten Pyrrolen mit Aminen oder Acylierungsmitteln.

EP 0 433 805 A1

## N-SUBSTITUIERTE 3-CYANO-4-PHENYL-PYRROLE

Die Erfindung betrifft neue N-substituierte 3-Cyano-4-phenyl-pyrrole, mehrere Verfahren zu ihrer Herstellung sowie ihre Verwendung als Schädlingsbekämpfungsmittel, insbesondere als Fungizide.

Es ist bereits bekannt, daß bestimmte 3-Cyano-4-phenyl-pyrrole, wie beispielsweise die Verbindung 4-Cyano-3-(2,3-dichlor-phenyl)-1-(N,N-dimethyl-aminomethyl)-pyrrol oder die Verbindung 4-Cyano-3-(2,3-dichlor-phenyl)-1-(N-methyl-N-furfuryl-aminomethyl)-pyrrol oder die Verbindung 4-Cyano-3-(2,3-dichlor-phenyl)-1-(N-ethyl-N-tetrahydrofurfurylaminomethyl)-pyrrol oder die Verbindung 1-Acetyl-3-cyano-4-(2,3-dichlorphenyl)-pyrrol oder die Verbindung 3-Cyano-4-(2,3-dichlorphenyl)-1-(ethoxycarbonyl)-pyrrol gute fungizide Eigenschaften besitzen (vgl. z.B. EP 133 247 oder EP 182 738 oder EP 281 731 oder DE 2 927 480 oder JP 8 051 066).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue N-substituierte 3-Cyano-4-phenyl-pyrrole der allgemeinen Formel (I),

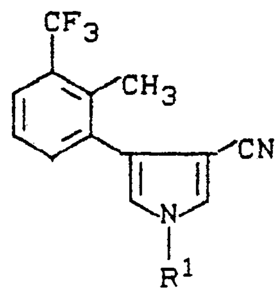

(I)

in welcher

$R^1$ für

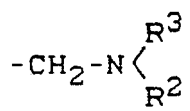 ; 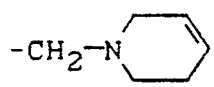 ; 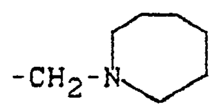 ;

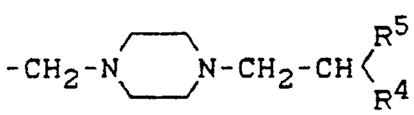 oder 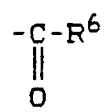 steht,

wobei

$R^2$

für gegebenenfalls substituiertes Alkyl, für Alkenyl, Alkinyl oder Cycloalkyl oder für jeweils gegebenenfalls substituiertes Aralkyl oder Aryl steht,

$R^3$

für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,

$R^4$

für Wasserstoff oder Alkyl steht,

$R^5$

für Cyano, Alkanoyl, Alkoxycarbonyl, Alkylaminocarbonyl, Dialkylaminocarbonyl, für Alkylsulfinyl, für Alkylsulfonyl, für Phenylsulfinyl oder für Phenylsulfonyl steht und

$R^6$
für Alkyl, Alkoxy, Alkoxyalkyl oder für Alkoxyalkyloxy steht,

gefunden.

Weiterhin wurde gefunden, daß man die neuen N-substituierten 3-Cyano-4-phenyl-pyrrole der allgemeinen Formel (I)

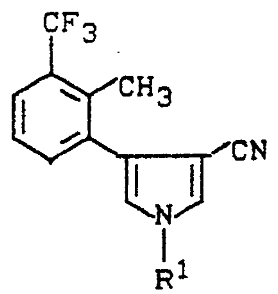

(I)

in welcher $R^1$
für

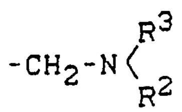 ; 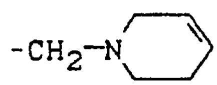 ; 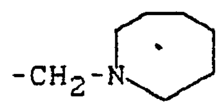 ;

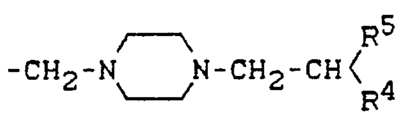 oder 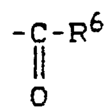 steht,

wobei

$R^2$
für gegebenenfalls substituiertes Alkyl, für Alkenyl, Alkinyl oder Cycloalkyl oder für jeweils gegebenenfalls substituiertes Aralkyl oder Aryl steht,

$R^3$
für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,

$R^4$
für Wasserstoff oder Alkyl steht,

$R^5$
für Cyano, Alkanoyl, Alkoxycarbonyl, Alkylaminocarbonyl, Dialkylaminocarbonyl, für Alkylsulfinyl, für Alkylsulfonyl, für Phenylsulfinyl oder für Phenylsulfonyl steht und

$R^6$
für Alkyl, Alkoxy, Alkoxyalkyl oder für Alkoxyalkyloxy steht,

erhält, wenn man

(a) das 3-(2-Methyl-3-trifluormethylphenyl)-4-cyano-pyrrol der Formel (II)

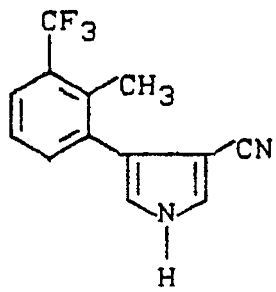

(II)

mit Formaldehyd und Aminen der Formel (III),

$$R^{1-1}-H \qquad (III)$$

in welcher
$R^{1-1}$ für einen Rest

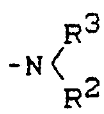 ; 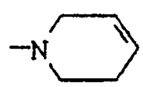 ; 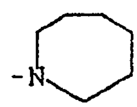

oder

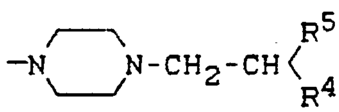 steht,

gegebenenfalls in Gegenwart eines Verdünnungsmittels und gegebenenfalls in Gegenwart eines Reaktionshilfsmittels umsetzt oder wenn man,
(b) 3-(2-Methyl-3-trifluormethylphenyl)-4-cyano-pyrrol-Derivate der Formel (IV),

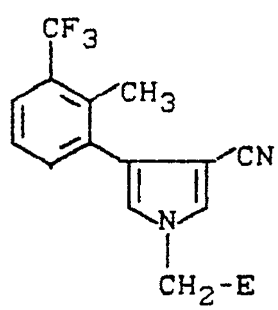

(IV)

in welcher

E
für eine elektronenanziehende Abgangsgruppe steht,

mit Aminen der Formel (III),

$$H\text{-}R^{1-1} \qquad (III)$$

in welcher

$R^{1-1}$

für einen Rest

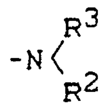 ; 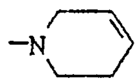 ; 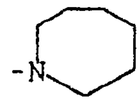

oder

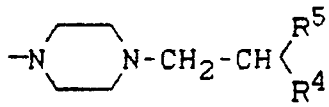 steht,

gegebenenfalls in Gegenwart eines Verdünnungsmittels und gegebenenfalls in Gegenwart eines Säurebindemittels umsetzt oder wenn man

(c) das 3-(2-Methyl-3-trifluormethylphenyl)-4-cyano-pyrrol der Formel (II),

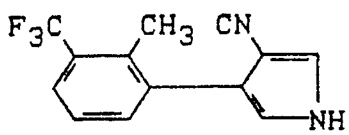

(II)

mit Acylierungsmitteln der Formel (V),

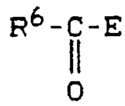

(V)

in welcher

$R^6$

die oben angegebene Bedeutung hat und

E

für eine elektronenanziehende Abgangsgruppe steht, gegebenenfalls in Gegenwart eines Verdünnungsmittels und gegebenenfalls in Gegenwart eines Reaktionshilfsmittels umsetzt.

Schließlich wurde gefunden, daß die neuen N-substituierten 3-Cyano-4-phenyl-pyrrole der allgemeinen Formel (I) eine gute Wirkung gegen Schädlinge, vor allem gegen Pilze besitzen.

Überraschenderweise zeigen die erfindungsgemäßen N-substituierten 3-Cyano-4-phenyl-pyrrole der allgemeinen Formel (I) eine erheblich bessere fungizide Wirksamkeit, als die aus dem Stand der Technik bekannten 3-Cyano-4-phenyl-pyrrole, wie beispielsweise die Verbindung 4-Cyano-3-(2,3-dichlor-phenyl)-1-(N,N-dimethylaminomethyl)-pyrrol oder die Verbindung 4-Cyano-3-(2,3-dichlor-phenyl)-1-(N-methyl-N-furfurylaminomethyl)-pyrrol oder die Verbindung 4-Cyano-3-(2,3-dichlor-phenyl)-1-(N-ethyl-N-tetrahydrofurfurylaminomethyl)-pyrrol oder die Verbindung 1-Acetyl-3-cyano-4-(2,3-dichlorphenyl)-pyrrol oder die Verbindung 3-Cyano-4-(2,3-dichlorphenyl)-1-(ethoxycarbonyl)-pyrrol, welche chemisch und wirkungsmäßig naheliegende Verbindungen sind.

Die erfindungsgemäßen N-substituierten 3-Cyano-4-phenyl-pyrrole sind durch die Formel (I) allgemein definiert. Bevorzugt sind Verbindungen der Formel (I), bei welchen

$R^1$
für einen Rest

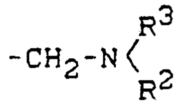 ; 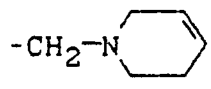 ; 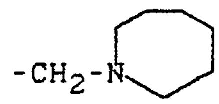 ;

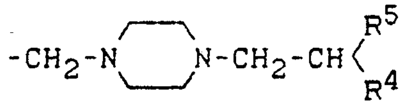 oder 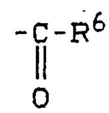 steht,

wobei

$R^2$
für gegebenenfalls substituiertes, geradkettiges oder verzweigtes Alkyl mit 1 bis 6 Kohlenstoffatomen steht, wobei als Substituenten infrage kommen: Cyano, Cycloalkyl mit 3 bis 7 Kohlenstoffatomen, jeweils geradkettiges oder verzweigtes Alkoxy, Alkylcarbonyl, Alkoxycarbonyl, Alkylaminocarbonyl, Dialkylaminocarbonyl, Alkylsulfinyl, Alkylsulfonyl oder Dialkylamino mit jeweils 1 bis 6 Kohlenstoffatomen in den einzelnen Alkylteilen; außerdem

$R^2$
für jeweils geradkettiges oder verzweigtes Alkenyl oder Alkinyl mit jeweils 3 bis 6 Kohlenstoffatomen, für Cycloalkyl mit 3 bis 7 Kohlenstoffatomen oder für jeweils gegebenenfalls einfach bis mehrfach, gleich oder verschieden im Phenylteil substituiertes Phenyl oder Benzyl steht, wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen,

$R^3$
für Heterocyclylalkyl mit 1 bis 6 Kohlenstoffatomen im Alkylteil, Heterocyclylalkenyl mit 3 bis 6 Kohlenstoffatomen im Alkenylteil, Heterocyclylalkinyl mit 3 bis 6 Kohlenstoffatomen im Alkinylteil oder Heterocyclyl steht, wobei Heterocyclyl jeweils für einen 5- bis 7-gliedrigen, gegebenenfalls einfach bis mehrfach, gleich oder verschieden substituierten, ungesättigen oder gesättigten Heterocyclus mit 1 bis 3 Heteroatomen, insbesondere Stickstoff, Sauerstoff oder Schwefel steht und wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen; außerdem $R^3$ für geradkettiges oder verzweigtes Dialkylaminoalkyl mit jeweils 1 bis 6 Kohlenstoffatomen in den einzelnen Alkylteilen steht, ferner für im Cycloalkylteil gegebenenfalls einfach bis mehrfach, gleich oder verschieden durch Halogen und geradkettigem oder verzweigtem Alkyl mit 1 bis 4 Kohlenstoffatomen substituiertes Cycloalkylalkyl mit 1 bis 6 Kohlenstoffatomen im geradkettigen oder verzweigten Alkylteil und 3 bis 7 Kohlenstoffatomen im Cycloalkylteil oder für im Phenylteil gegebenenfalls einfach bis mehrfach, gleich oder verschieden durch Cyano, Halogen, jeweils geradkettigem oder verzweigtem Alkyl oder Alkoxy mit jeweils 1 bis 4 Kohlenstoffatomen substituiertes Phenylethyl steht,

$R^4$
für Wasserstoff oder für geradkettiges oder verzweigtes Alkyl mit 1 bis 6 Kohlenstoffatomen steht,

$R^5$
für Cyano oder für jeweils geradkettiges oder verzweigtes Alkanoyl, Alkoxycarbonyl, Alkylaminocarbonyl, Dialkylaminocarbonyl, Alkylsulfinyl oder Alkylsulfonyl mit jeweils 1 bis 6 Kohlenstoffatomen in den einzelnen Alkylteilen oder für Phenylsulfinyl oder Phenylsulfonyl steht und

$R^6$
für jeweils geradkettiges oder verzweigtes Alkyl, Alkoxy, Alkoxyalkyl oder Alkoxyalkyloxy mit jeweils 1 bis 6 Kohlenstoffatomen in den einzelnen Alkylteilen steht.

Besonders bevorzugt sind Verbindungen der Formel (I), bei welchen
$R^1$
für einen Rest

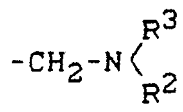 ; 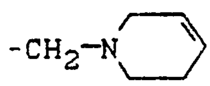 ; 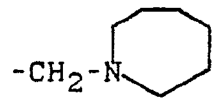 ;

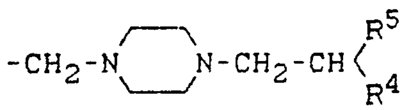 oder 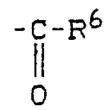 steht,

wobei

$R^2$
für jeweils gegebenenfalls substituiertes Methyl, Ethyl oder n- oder i-Propyl sowie n-, i- oder s-Butyl steht, wobei als Substituenten jeweils infrage kommen: Cyano, Methoxy, Ethoxy, Methylcarbonyl, Ethylcarbonyl, Methoxycarbonyl, Ethoxycarbonyl, Methylaminocarbonyl, Dimethylaminocarbonyl, Ethylaminocarbonyl, Diethylaminocarbonyl, Methylsulfinyl, Methylsulfonyl, Dimethylamino, Diethylamino oder Dipropylamino, Dibutylamino oder Cyclohexyl; außerdem $R^2$ für Allyl, n- oder i-Butenyl, Propargyl, n- oder i-Butinyl, für Cyclopropyl, Cyclopentyl oder Cyclohexyl, oder für jeweils gegebenenfalls ein- bis dreifach, gleich oder verschieden im Phenylteil substituiertes Benzyl oder Phenyl steht, wobei als Substituenten infrage kommen: Fluor, Chlor, Brom, Cyano, Methyl, Ethyl, n-oder i-Propyl, n-, i-oder s-Butyl, Methoxy, Ethoxy, Trifluormethyl, Trifluormethoxy, Acetyl, Methoxycarbonyl oder Ethoxycarbonyl,

$R^3$
für jeweils gegebenenfalls im Heterocyclylteil ein- oder zweifach, gleich oder verschieden substituiertes Heterocyclylmethyl, Heterocyclylethyl oder Heterocyclylpropyl steht, wobei als Heterocyclylrest jeweils einer der folgenden Reste infrage kommt

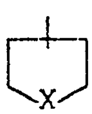 ; 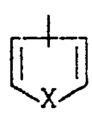 ; 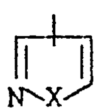 ; 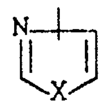 ; 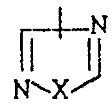 ;

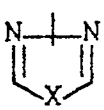 ; 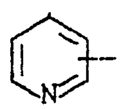 ; 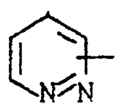 ; 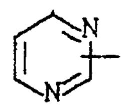 ; 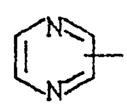 ;

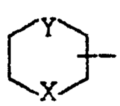 , 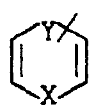 , oder 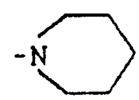

wobei X jeweils für Sauerstoff, Schwefel oder eine NH-Gruppe steht und Y jeweils für Sauerstoff, Schwefel, eine NH-Gruppe oder eine $CH_2$-Gruppe steht und wobei als Substituenten jeweils infrage kommen: Fluor, Chlor, Brom, Cyano, Methyl, Ethyl, n- oder i-Propyl, n-, i-, s- oder t-Butyl, Methoxy, Ethoxy, Trifluormethyl, Trifluormethoxy, Acetyl, Methoxycarbonyl oder Ethoxycarbonyl; außerdem $R^3$ für Dimethylamino/ethyl,

Diethylaminoethyl, Dimethylaminopropyl, Diethylaminopropyl, Dipropylaminopropyl, Dibutylaminopropyl, Dibutylaminobutyl, Dibutylaminoethyl, Dipropylaminoethyl, Dimethylaminobutyl, Diethylaminobutyl, Dimethylaminopentyl, Diethylaminopentyl, für jeweils gegebenenfalls ein- bis dreifach, gleich oder verschieden durch Methyl, Ethyl und Isopropyl substituiertes Cyclohexylmethyl oder Cyclohexylethyl oder für gegebenenfalls ein- bis dreifach, gleich oder verschieden durch Fluor, Chlor, Brom, Cyano, Methyl, Ethyl, n-oder i-Propyl, n-, i-, s-oder t-Butyl, Methoxy, Ethoxy, n- oder i-Propoxy und n-, i-, s- oder t-Butoxy substituiertes Phenethyl steht,

$R^4$
für Wasserstoff, Methyl, Ethyl, n- oder i-Propyl sowie n-, i-, s- oder t-Butyl steht,

$R^5$
für Cyano, Acetyl, Propionyl, Methoxycarbonyl, Ethoxycarbonyl, Methylaminocarbonyl, Dimethylaminocarbonyl, Ethylaminocarbonyl, Diethylaminocarbonyl, Dipropylaminocarbonyl, Dibutylaminocarbonyl, Methylsulfinyl, Ethylsulfinyl, Methylsulfonyl, Ethylsulfonyl, Phenylsulfinyl oder Phenylsulfonyl steht und

$R^6$
für jeweils geradkettiges oder verzweigtes Alkyl, Alkoxy, Alkoxyalkyl oder Alkoxyalkyloxy mit jeweils 1 bis 4 Kohlenstoffatomen in den einzelnen Alkylteilen steht.

Ganz besonders bevorzugt sind Verbindungen der Formel (I), bei welchen
$R^1$
für einen Rest

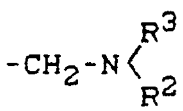 ; 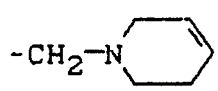 ; 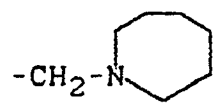 ;

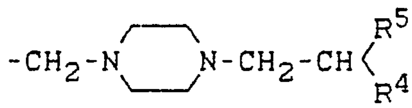 oder 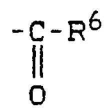 steht,

wobei

$R^2$
für Methyl, Ethyl, n- oder i-Propyl, n-, i- oder s-Butyl, Allyl, Propargyl, für Cyclohexyl, Cyclopentyl, Cyclopropyl, Cyclohexylmethyl, Phenyl, Benzyl oder für Cyanethyl steht,

$R^3$
für jeweils gegebenenfalls ein- bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

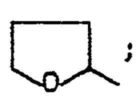 ; 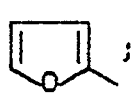 ; 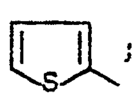 ; 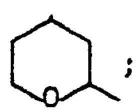 ; 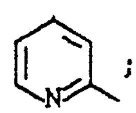 ;

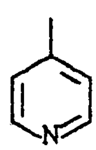 oder 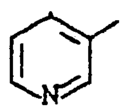 ;

oder für jeweils gegebenenfalls ein- bis dreifach, gleich oder verschieden durch Methyl und Ethyl

substituiertes 2-Heterocyclylethyl, 2-Heterocyclylpropyl oder 3-Heterocyclylpropyl steht, wobei als Heterocyclylreste infrage kommen:

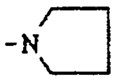 ; 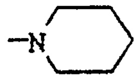 ; 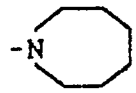 ; 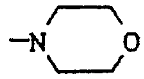 ;

außerdem für Diethylaminoethyl, Dimethylaminopropyl, Diethylaminopropyl, Dibutylaminopropyl, Diethylaminopentyl, Cyclohexylmethyl oder Phenethyl steht,

$R^4$
für Wasserstoff steht,

$R^5$
für Cyano, Acetyl, Propionyl, Methoxycarbonyl, Ethoxycarbonyl, Dimethylaminocarbonyl, Methylsulfinyl, Methylsulfonyl, Phenylsulfinyl oder Phenylsulfonyl steht und

$R^6$
für Methyl, Ethyl, Methoxy, Ethoxy, Methoxymethyl, Ethoxymethyl, Methoxymethyloxy, Methoxyethyloxy oder Ethoxyethyloxy steht.

Insbesonders bevorzugt sind Verbindungen der Formel (I), bei welchen
$R^1$
für einen Rest

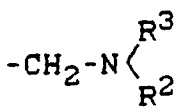 oder 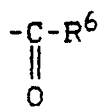 steht,

wobei

$R^2$
für Cyanethyl steht,

$R^3$
für jeweils gegebenenfalls ein- bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

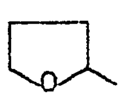 ; 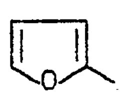 ; 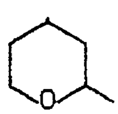 ; 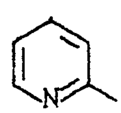 ;

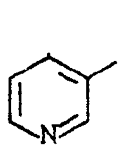 oder 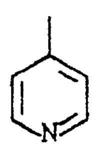 ;

oder für jeweils gegebenenfalls ein- bis dreifach, gleich oder verschieden durch Methyl und Ethyl substituiertes 2-Heterocyclylethyl, 2-Heterocyclylpropyl oder 3-Heterocyclylpropyl steht, wobei als Heterocyclylreste infrage kommen:

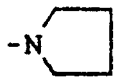 ; 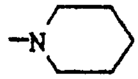 ; 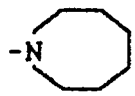 oder 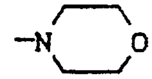 ;

außerdem für Diethylaminoethyl, Dimethylaminopropyl, Diethylaminopropyl, Dibutylaminopropyl Diethylaminopentyl, Cyclohexylmethyl oder Phenethyl steht und

$R^6$

für Methyl, Ethyl, Methoxy, Ethoxy, Methoxymethyl, Ethoxymethyl, Methoxyethyloxy oder Ethoxyethyloxy steht.

Insbesondere bevorzugt sind daneben auch Verbindungen der Formel (I), bei welchen

$R^1$

für einen Rest

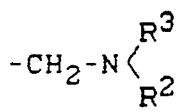 oder 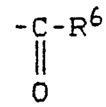 steht,

wobei

$R^2$

für Methyl, Ethyl, n- oder i-Propyl, n-, i- oder s-Butyl, Allyl, Propargyl, Cyclopropyl, Cyclopentyl, Cyclohexyl, Cyclohexylmethyl, Phenyl, Benzyl oder Cyanoethyl steht,

$R^3$

für gegebenenfalls ein- bis dreifach, gleich oder verschieden durch Chlor und Methyl substituiertes Heterocyclylmethyl steht, wobei als Heterocyclylreste infrage kommen:

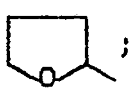 ; 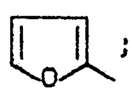 ; 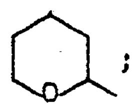 ; 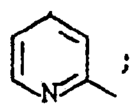 ; 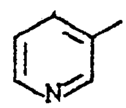

oder 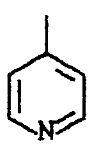 und

$R^6$

für Methyl, Ethyl, Methoxy, Ethoxy, Methoxymethyl, Ethoxymethyl, Methoxyethyloxy oder Ethoxyethyloxy steht.

Im einzelnen seien außer den bei den Herstellungsbeispielen genannten Verbindungen die folgenden N-substituierten 3-Cyano-4-phenyl-pyrrole der allgemeinen Formel (I) genannt:

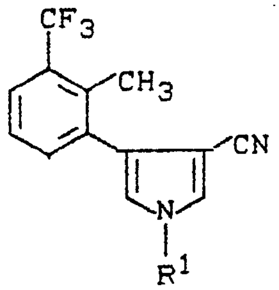 (I)

| $R^1$ | $R^1$ |
|---|---|
| 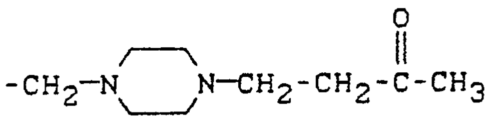 | 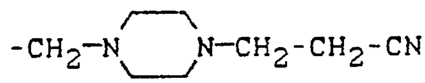 |
| 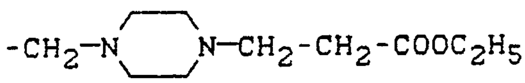 | 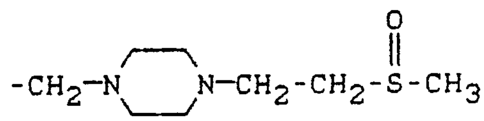 |
| 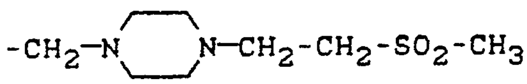 | 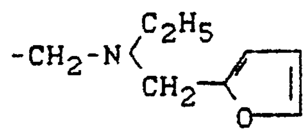 |

| $R^1$ | $R^1$ |
|---|---|
| 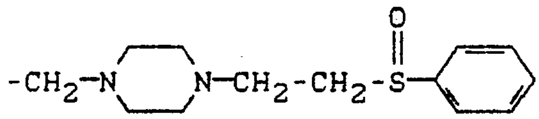 | 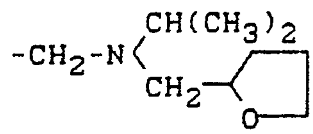 |
| 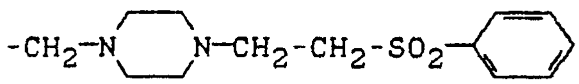 | 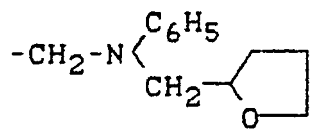 |
| 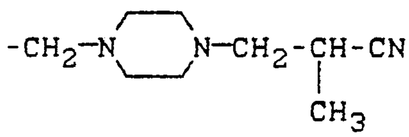 | 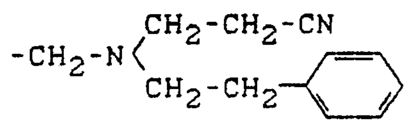 |
| 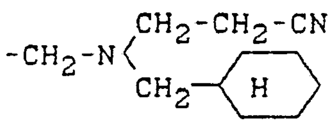 | 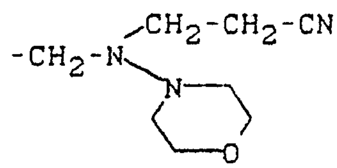 |
| 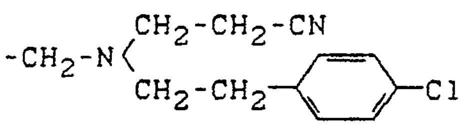 | 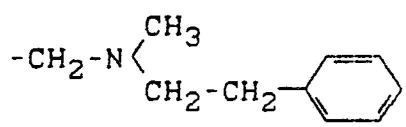 |
| 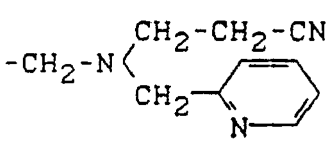 | 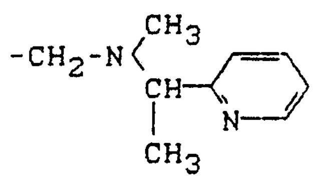 |
| 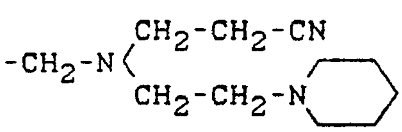 | 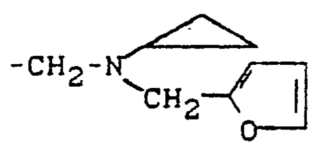 |
| 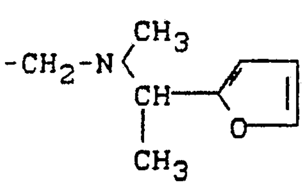 | 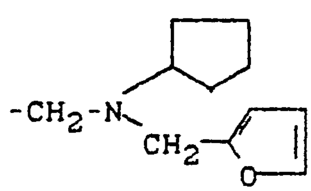 |

| $R^1$ | $R^1$ |
|---|---|
| 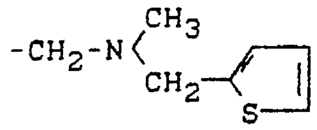 | 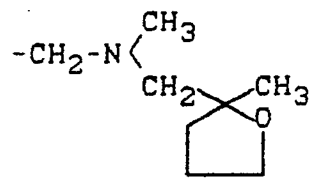 |
| 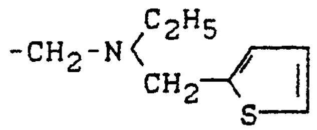 | 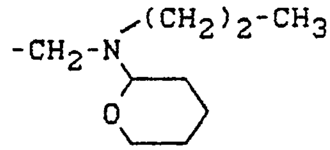 |
| 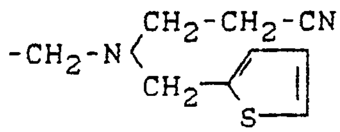 | 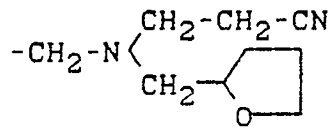 |
| 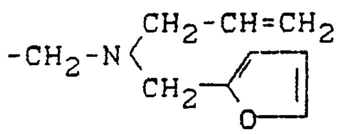 | 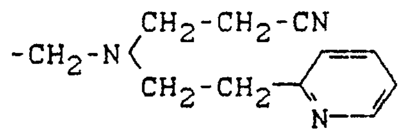 |
| 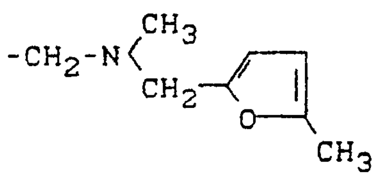 | 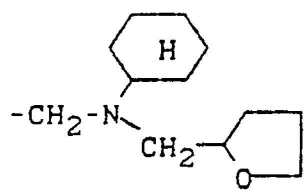 |
| 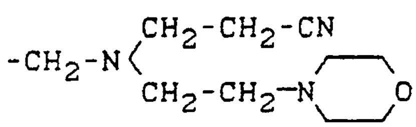 | 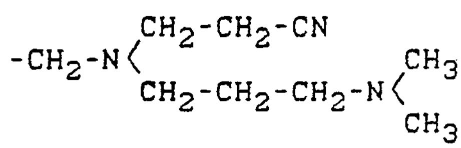 |
| 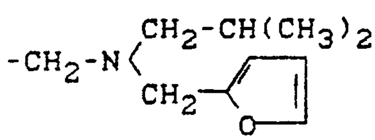 | 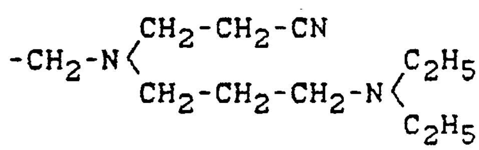 |
| 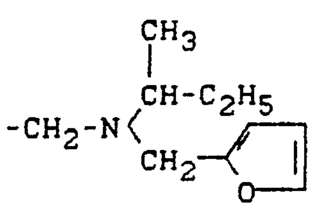 | 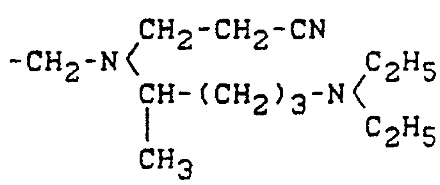 |

| $R^1$ | $R^1$ |
|---|---|
| 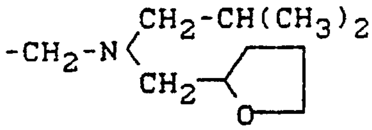 | 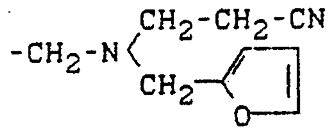 |
| 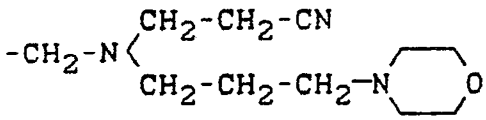 | 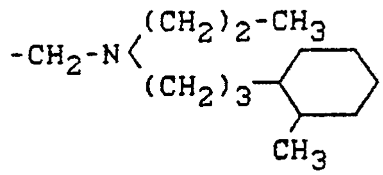 |
| 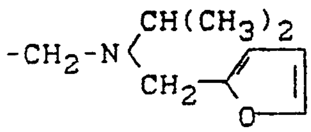 | 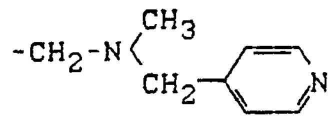 |
| 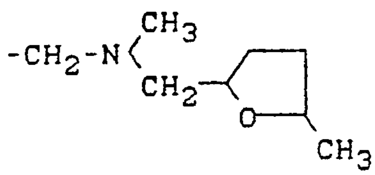 | 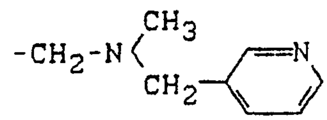 |
| 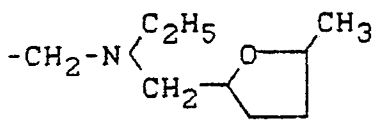 | |
| 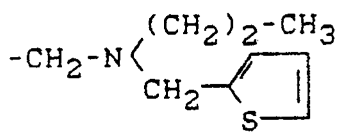 | |
| 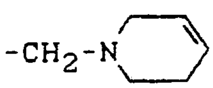 | |
| 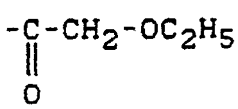 | |
| 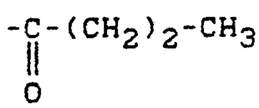 | |

| $R^1$ | $R^1$ |
|---|---|
| $-\underset{\underset{O}{\|}}{C}-CH_2-O-(CH_2)_2-CH_3$ | $-\underset{\underset{O}{\|}}{C}-C_2H_5$ |
| $-\underset{\underset{O}{\|}}{C}-CH(CH_3)_2$ | $-\underset{\underset{O}{\|}}{C}-OCH_3$ |
| $-\underset{\underset{O}{\|}}{C}-OC_2H_5$ | |
| $-\underset{\underset{O}{\|}}{C}-O-(CH_2)_2-CH_3$ | $-\underset{\underset{O}{\|}}{C}-CH_3$ |
| $-\underset{\underset{O}{\|}}{C}-O-CH(CH_3)_2$ | $-\underset{\underset{O}{\|}}{C}-CH_2-OCH_3$ |
| $-\underset{\underset{O}{\|}}{C}-O-\underset{\underset{CH_3}{\|}}{CH}-C_2H_5$ | |
| | $-\underset{\underset{O}{\|}}{C}-OCH_2CH_2-OCH_3$ |

Alle aliphatischen Reste können geradkettig oder verzweigt sein, ob es ausdrücklich angegeben wurde oder nicht.

Wenn nicht anders angegeben, können alle Ringsysteme gleich oder verschieden, bevorzugt 1-5-fach, insbesondere 1-3-fach substituiert sein.

Die aliphatischen Reste können ebenfalls 1-5-fach, insbesondere 1-3-fach, ganz besonders bevorzugt ein- oder zweifach substituiert sein.

Verwendet man beispielsweise 3-Cyano-4-(2-methyl-3-trifluormethylphenyl)-pyrrol, Formaldehyd und N-Methyl-N-(2-tetrahydrofurylmethyl)-amin als Ausgangsstoffe, so läßt sich der Reaktionsablauf des erfindungsgemäßen Verfahrens (a) durch das folgende Formelschema darstellen:

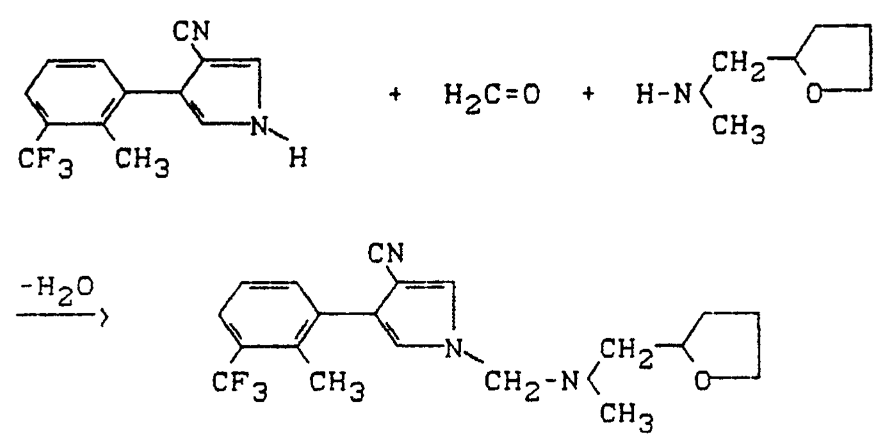

Verwendet man beispielsweise 1-Chlormethyl-3-cyano-4-(2-methyl-3-trifluormethyl)-pyrrol und N-Ethyl-N-(2-furylmethyl)-amin als Ausgangsstoffe, so läßt sich der Reaktionsablauf des erfindungsgemäßen Verfahrens (b) durch das folgende Formelschema darstellen:

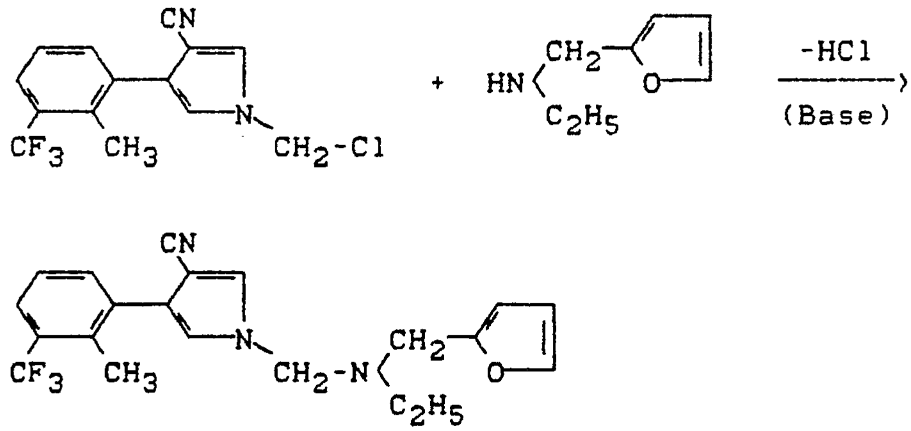


Verwendet man beispielsweise 3-Cyano-4-(2-methyl-3-trifluormethylphenyl)-pyrrol und Chlorameisensäureethylester als Ausgangsstoffe, so läßt sich der Reaktionsablauf des erfindungsgemäßen Verfahrens (c) durch das folgende Formelschema darstellen:

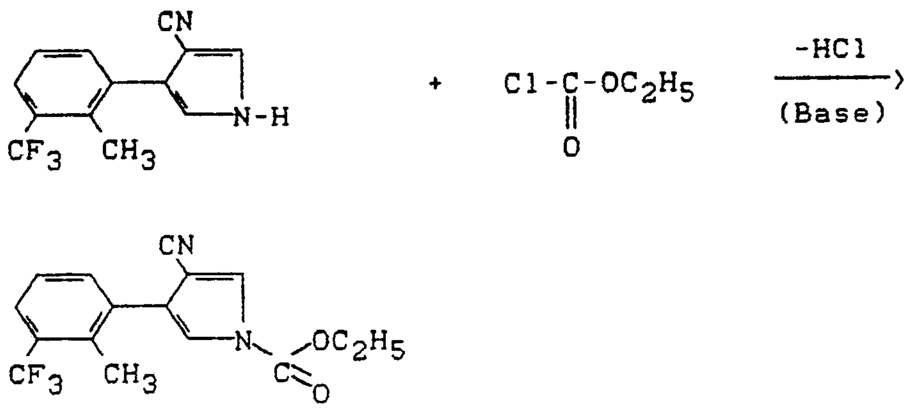


Das zur Durchführung des erfindungsgemäßen Verfahrens (a) und (c) als Ausgangsverbindung benötigte 3-Cyano-4-(2-methyl-3-trifluormethylphenyl)-pyrrol ist durch die Formel (II) definiert.

Das 3-Cyano-4-(2-methyl-3-trifluormethylphenyl)-pyrrol der Formel (II) ist noch nicht bekannt. Es ist jedoch Gegenstand einer eigenen vorgängigen aber nicht vorveröffentlichten deutschen Patentanmeldung und erhältlich nach den dort beschriebenen Verfahren, beispielsweise wenn man 2-Methyl-3-trifluormethyl-zimtsäurenitril der Formel (VI)

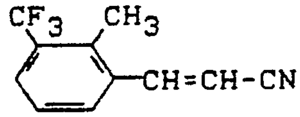

(VI)

mit p-Toluolsulfonylmethylisocyanid der Formel (VII)

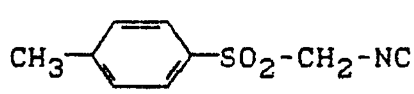

(VII)

in Gegenwart einer Base wie beispielsweise Natriumhydrid und gegebenenfalls in Gegenwart eines

Verdünnungsmittels wie beispielsweise Tetrahydrofuran bei Temperaturen zwischen -20 °C und +50 °C umsetzt (vgl. auch die Herstellungsbeispiele).

2-Methyl-3-trifluormethyl-zimtsäurenitril der Formel (VI) ist neu. Man erhält es jedoch in Analogie zu bekannten Verfahren (vgl. z.B. DE-OS 2 927 480), beispielsweise wenn man 2-Methyl-3-trifluormethylanilin der Formel (VIII)

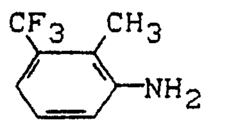

(VIII)

zunächst in einer ersten Stufe mit Acrylnitril unter üblichen Diazotierungsbedingungen, beispielsweise in Gegenwart von Natriumnitrit und Salzsäure, und in Gegenwart eines geeigneten Metallsalz-Katalysators, wie beispielsweise Kupfer-II-chlorid oder Kupfer-II-oxid, und gegebenenfalls in Gegenwart eines geeigneten Verdünnungsmittels, wie beispielsweise Aceton oder Wasser, bei Temperaturen zwischen -20 °C und 50 °C umsetzt ("Meerwein-Arylierung"; vgl. hierzu auch Organic Reactions 11, 189 [1960]; Organic Reactions 24, 225 [1976] oder C. Ferri "Reaktionen der organischen Synthese" S. 319, Thieme Verlag Stuttgart 1978) und dann in einer 2. Stufe das so erhältliche α-Chlor-β-(2-methyl-3-trifluormethylphenyl)-propionitril der Formel (IX)

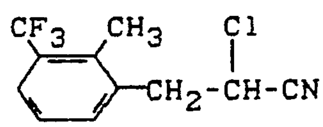

(IX)

mit Basen, wie beispielsweise Triethylamin oder Diazabicycloundecen, in üblicher Art und Weise gegebenenfalls in Gegenwart eines Verdünnungsmittels, wie beispielsweise Tetrahydrofuran, bei Temperaturen zwischen 0 °C und 50 °C dehydrohalogeniert.

2-Methyl-3-trifluormethylanilin der Formel (VII) ist bekannt (vgl. z.B. EP 295 674; DE 3 726 891, US 4 209 464; US 4 172 095 und US 4 132 737). p-Toluolsulfonylmethylisocyanid der Formel (VII) ist ebenfalls bekannt (vgl. z.B. Synthesis 1985, 400-402; Tetrahedron Lett. 1972, 2367-2368).

Die zur Durchführung der erfindungsgemäßen Verfahren (a) und (b) weiterhin als Ausgangsstoffe benötigten Amine sind durch die Formel (III) allgemein definiert. In dieser Formel (III) steht $R^{1-1}$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Amine der Formel (III) sind allgemein bekannte Verbindungen der organischen Chemie oder erhältlich in Analogie zu bekannten Verfahren (vgl. z.B. GB 1 031 916 vom 02.06.1966; Org. Magnet. Res. 7, 488-495 [1975]; Kogyo Kagaku Zasshi 63, 1593-1597 [1960] bzw. CA 60: 10 542 f).

Alternativ zur Herstellung der Vorprodukte der Formel (II) mit Hilfe der im vorangegangenen beschriebenen Herstellungsverfahren sind verschiedene weitere Herstellungsverfahren zur Herstellung der Vorprodukte der Formel (II) denkbar.

So erhält man 3-(2-Methyl-3-trifluormethylphenyl)-4-cyano-pyrrol der Formel (II) beispielsweise auch, wenn man α-Cyanozimtsäureester in Gegenwart von Basen und in Gegenwart von Kupfer-(II)-Salzen mit p-Toluol-sulfonylmethylisocyanid umsetzt (vgl. J 61 030 571 oder J 61 200 984) oder wenn man α-substituierte Zimtsäurenitrile in Gegenwart von Natriumhydrid mit Isocyanoessigsäureestern cyclisiert, die so erhältlichen Pyrrol-2-carbonsäureester mit Basen verseift und anschließend thermisch decarboxyliert (vgl. JP 59 212 468) oder wenn man Phenacylaminderivate mit geeignet substituierten Acrylnitril-Derivaten umsetzt (vgl. EP 174 910) oder wenn man 3-Trifluormethyl-4-phenyl-pyrrole mit Ammoniak bei erhöhter Temperatur und erhöhtem Druck umsetzt (vgl. EP 182 738) oder wenn man 3-Cyano-4-phenyl-$\Delta^2$-pyrroline in Gegenwart von Kupfer-II-Salzen oder Eisen-III-salzen oxidiert (vgl. EP 183 217) oder wenn man α-Cyanoacrylsäurederivate mit Isocyanoessigsäureestern in Gegenwart einer Base umsetzt und die so erhältlichen $\Delta^2$-Pyrrolin-2-carbonsäurederivate in einer 2. Stufe in Gegenwart einer Base und in Gegenwart eines Metallsalzkatalysators oxidativ decarboxyliert (vgl. Deutsche Patentanmeldung P 3 718 375 vom 02.06.87).

Die 3-(2-Methyl-3-trifluormethylphenyl)-4-cyano-pyrrol-Derivate der Formel (IV) gemäß Verfahrensvariante (b) sind ebenfalls noch nicht bekannt. Man erhält sie jedoch nach bekannten Verfahren (vgl. EP 133 247),

wenn man 3-(2-Methyl-3-trifluormethylphenyl)-4-cyano-pyrrol der Formel (II),

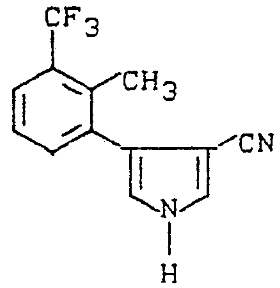

(II)

mit Formaldehyd gegebenenfalls in Gegenwart eines üblichen Verdünnungsmittels wie beispielsweise Tetrahydrofuran und gegebenenfalls in Gegenwart eines Halogenierungsmittels wie beispielsweise Thionylchlorid oder Phosphoroxychlorid sowie gegebenenfalls in Gegenwart eines Säurebindemittels wie beispielsweise Triethylamin oder Diazabicycloundecen (DBU) bei Temperaturen zwischen 20° C und 160° C umsetzt.

Die zur Durchführung des erfindungsgemäßen Verfahrens (c) weiterhin als Ausgangsstoffe benötigten Acylierungsmittel sind durch die Formel (V) allgemein definiert. In dieser Formel (V) steht $R^6$ vorzugsweise für diejenigen Reste, die bereits im Zusammenhang mit der Beschreibung der erfindungsgemäßen Stoffe der Formel (I) als bevorzugt für diesen Substituenten genannt wurden.

E

steht vorzugsweise für Halogen, insbesondere für Chlor oder Brom, oder für einen Anhydridrest der Formel

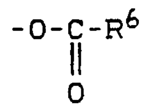

wobei

$R^6$

die oben angegebene Bedeutung hat.

Die Acylierungsmittel der Formel (V) sind allgemein bekannte Verbindungen der organischen Chemie.

Als Verdünnungsmittel zur Durchführung des erfindungsgemäßen Verfahrens (a) kommen inerte organische Lösungsmittel oder wässrige Systeme infrage. Vorzugsweise verwendet man protische Lösungsmittel, beispielsweise Alkohole, wie Methanol, Ethanol oder Propanol oder Carbonsäuren, wie Ameisensäure, Essigsäure oder Propionsäure oder deren Gemische mit Wasser. Es ist auch möglich, das erfindungsgemäße Verfahren (a) in aprotischen Lösungsmitteln duchzuführen. Hierzu gehören insbesondere aliphatische oder aromatische, gegebenenfalls halogenierte Kohlenwasserstoffe, wie beispielweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (a) wird gegebenenfalls in Gegenwart eines geeigneten Reaktionshilfmittels durchgeführt. Hierzu eignen sich entweder katalytische bis äquimolare Mengen einer organischen oder anorganischen Säure oder entsprechende Mengen einer geeigneten Base.

Als saure Reaktionshilfsmittel kommen insbesondere anorganische Mineralsäuren, wie Phosphorsäure, Schwefelsäure, Salpetersäure, Chlorwasserstoffsäure oder Bromwasserstoffsäure oder organische Säuren, wie Ameisensäure, Essigsäure, Propionsäure, Methansulfonsäure, Benzolsulfonsäure oder Toluolsulfonsäure infrage.

Als basische Reaktionshilfsmittel kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan

(DABCO), Diazabicyclononen(DBN) oder Diazabicycloundecen(DBU).

Es ist jedoch auch möglich, das als Reaktionspartner verwendete Amin der Formel (III) in entsprechendem Überschuß gleichzeitig als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 120 °C, vorzugsweise bei Temperaturen zwischen 20 °C und 90 °C.

Zur Durchführung des erfindungsgemäßen Verfahrens (a) setzt man pro Mol an 3-(2-Methyl-3-trifluormethylphenyl)-4-cyano-pyrrol der Formel (II) im allgemeinen 1,0 bis 2,0 Mol, vorzugsweise 1,0 bis 1,5 Mol an Amin der Formel (III) und 1,0 bis 2,0 Mol, vorzugsweise 1,0 bis 1,5 Mol an Formaldehyd ein. Der Formaldehyd wird entweder in Form einer wässrigen Lösung, als Paraformaldehyd oder als 1,3,5-Trioxan eingesetzt. Vorzugsweise verwendet man eine wässrige Lösung. Die Reaktionsdurchführung, Aufarbeitung und Isolierung der Reaktionsprodukte der Formel (I) erfolgt in Analogie zu bekannten Verfahren (vgl. EP 133 247).

Als Verdünnungsmittel zur Durchführung des erfindungsgemäßen Verfahrens (b) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) wird gegebenenfalls in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen(DBN) oder Diazabicycloundecen(DBU).

Es ist jedoch auch möglich, das als Reaktionspartner in Frage kommende Amin der Formel (III) in entsprechendem Überschuß gleichzeitig als Reaktionshilfsmittel einzusetzen.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 100 °C, vorzugsweise bei Temperaturen zwischen 20 °C und 60 °C.

Zur Durchführung des erfindungsgemäßen Verfahrens (b) setzt man pro Mol an 3-(2-Methyl-3-trifluormethylphenyl)-4-cyano-pyrrol der Formel (IV) im allgemeinen 1,0 bis 2,0 Mol, vorzugsweise 1,0 bis 1,5 Mol an Amin der Formel (III) und gegebenenfalls 1,0 bis 2,0 Mol, vorzugsweise 1,0 bis 1,5 Mol an Säurebindemittel ein. Die Reaktionsdurchführung, Aufarbeitung und Isolierung der Reaktionsprodukte der Formel (I) erfolgt in Analogie zu bekannten Verfahren.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (c) wird vorzugsweise in Gegenwart eines geeigneten Reaktionshilfsmittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen - 20 °C und 150 °C, vorzugsweise bei Temperaturen zwischen 0 °C und 80 °C.

Zur Durchführung des erfindungsgemäßen Verfahrens (c) setzt man pro Mol an 3-(2-Methyl-3-trifluormethyl-phenyl)-4-cyano-pyrrol der Formel (II) im allgemeinen 1.0 bis 5.0 Mol, vorzugsweise 1.0 bis 1.5 Mol an Acylierungsmittel der Formel (III) und gegebenenfalls 1.0 bis 5.0 Mol, vorzugsweise 1.0 bis 1.5 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind z.B. für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg protektiv zur Bekämpfung von Krankheiten im Obst- und Gemüseanbau, wie beispielsweise gegen den Erreger des Bohnengrauschimmels (Botrytis cinerea), oder zur Bekämpfung von Reiskrankheiten, wie beispielsweise gegen den Erreger der Reisfleckenkrankheit (Pyricularia oryzae) oder auch zur Bekämpfung von Getreidekrankheiten, wie beispielsweise gegen Erysiphe an Weizen oder Cochliobolus sativus an Gerste oder Pyrenophora teres an Gerste einsetzen.

Darüberhinaus besitzen die erfindungsgemäßen Wirkstoffe eine gute fungizide in-vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen,

Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1

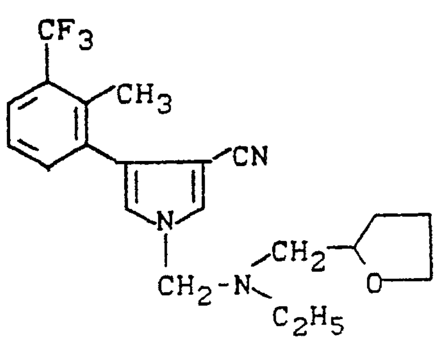

(Verfahren (a))

Zu einer Mischung aus 3,0 g (0,012 Mol) 3-(2-Methyl-3-trifluormethylphenyl)-4-cyano-pyrrol, 0,78 g (0,0126 Mol) 48,5%iger Formaldehydlösung in Methanol und 0,1 ml Eisessig in 10 ml Ethanol gibt man 1,62 g (0,0126 Mol) N-Ethyltetrahydrofurfurylamin zu. Man erwärmt, bis eine klare Lösung entsteht, läßt 16 Stunden bei Raumtemperatur nachrühren und engt ein.

Man erhält 4,6 g (98% der Theorie) 4-Cyano-3-(2-methyl-3-trifluormethylphenyl)-1-(N-ethyl-N-tetrahydro-furfurylaminomethyl) -pyrrol.

$^1$H-NMR ($CDCl_3$, 300 MHz)δ in ppm (3,7-4,2, m, (3H); 4,8-5,1 q, (1H); 6,7 ppm, s, (1H)).

Beispiel 2

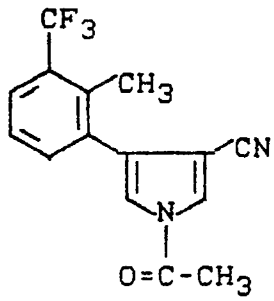

(Verfahren (c))

Eine Lösung aus 3,0 g (0,012 Mol) 3-(2-Methyl-3-trifluormethylphenyl)-4-cyano-pyrrol in 120 ml Methylenchlorid/Tetrahydrofuran 5:1 wird mit 1,28 g (0,0126 Mol) Essigsäureanhydrid, 1,28 g (0,0126 Mol) Triethylamin und 0,2 g Dimethylaminopyridin versetzt. Nach 16 Stunden Rühren bei Raumtemperatur gibt man die Reaktionsmischung auf Wasser, trennt die Phasen, wäscht die organische Phase nochmals mit Wasser, trocknet über Natriumsulfat und engt ein.

Man erhält 3,2 g (91% der Theorie) 1-Acetyl-3-cyano-4-(2-methyl-3-trifluormethylphenyl)-pyrrol vom Schmelzpunkt 163° C.

Beispiel 3

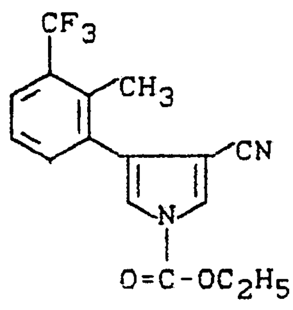

(Verfahren (c))

Zu einer Suspension aus 0,43 g (0,0144 Mol) Natriumhydrid (80%ig in Mineralöl) in 10 ml Dimethoxyethan wird unter Argon bei Raumtemperatur 3,0 g (0,012 Mol) 3-(2-Methyl-3-trifluormethylphenyl)-4-cyano-pyrrol in 50 ml Dimethoxyethan/Tetrahydrofuran 4:1 getropft und die Reaktionsmischung 2 Stunden bei Raumtemperatur gerührt. Anschließend tropft man 2,3 g (0,0209 Mol) Chlorameisensäureethylester in 10 ml Dimethoxyethan zu, rührt weitere 3 Stunden bei Raumtemperatur, hydrolysiert mit 200 ml Wasser und extrahiert das Produkt mit Essigester.

Nach Waschen der Essigesterphase mit Wasser, Trocknen über Natriumsulfat und Einengen erhält man 3,8 g (97% der Theorie) 1-Ethoxycarbonyl-3-cyano-4-(2-methyl-3-trifluormethylphenyl)-pyrrol vom Schmelzpunkt 60-62° C.

In analoger Weise zu Beispiel 1 bis 3 und unter Berücksichtigung der Angaben in den Beschreibungen der erfindungsgemäßen Verfahren werden die nachfolgend in Tabelle II aufgeführten Endprodukte der Formel (I)

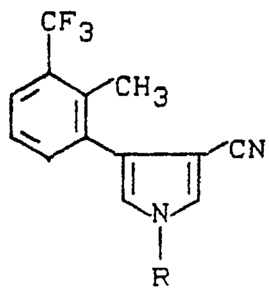

(I)

erhalten:

Tabelle II:

| Bsp.-Nr. | R | Physik. Konstanten |
|---|---|---|
| 4 | 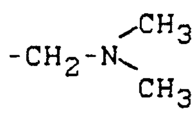 | Fp: 66 - 70° C |
| 5 | 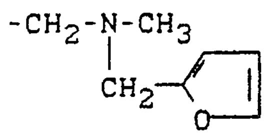 | $^1$H-NMR ($CDCl_3$)*) δ = (4,7, s, (1H); 6,3, s, (1H); 6,4, s, (1H); 6,7, s, (1H))ppm |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Anwendungsbeispiele

In den folgenden Anwendungsbeispielen wurden die nachstehend aufgeführten Verbindungen als Vergleichssubstanzen eingesetzt:

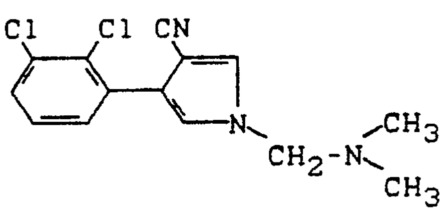

(A)

4-Cyano-3-(2,3-dichlor-phenyl)-1-(N,N-dimethylaminomethyl)-pyrrol (bekannt aus EP 133 247 und EP 182 738).

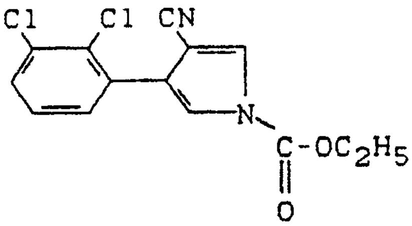

(B)

3-Cyano-4-(2,3-dichlorphenyl)-1-(ethoxycarbonyl)-pyrrol (bekannt aus JP 8 051 066).

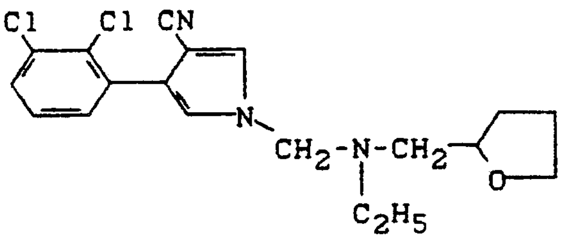

(C)

4-Cyano-3-(2,3-dichlor-phenyl)-1-(N-ethyl-N-tetrahydrofurfurylaminomethyl)-pyrrol (bekannt aus EP 281 731).

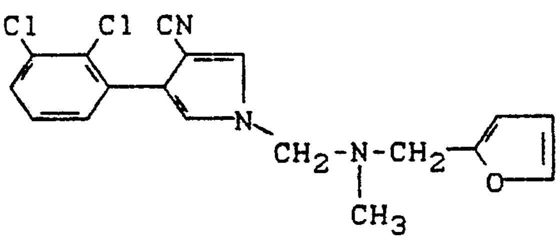

(D)

4-Cyano-3-(2,3-dichlor-phenyl)-1-(N-methyl-N-furfurylaminomethyl)-pyrrol (bekannt aus EP 281 731).

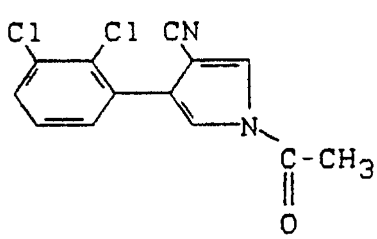

(E)

1-Acetyl-3-cyano-4-(2,3-dichlorphenyl)-pyrrol (bekannt aus DE-OS 2 927 480).

Beispiel A

Erysiphe-Test (Weizen)/protektiv

| | | |
|---|---|---|
| Lösungsmittel: | 100 | Gewichtsteile Dimethylformamid |
| Emulgator: | 0,25 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20° C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen 3 und 4.

Beispiel B

Cochliobolus sativus-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20° C und 100% relativer Luftfeuchtigkeit in einer Inkubationskabine.

Die Pflanzen werden in einem Gewächshaus bei einer Temperatur von ca. 20° C und einer relativen Luftfeuchtigkeit von ca. 80% aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen 1, 3 und 5.

Beispiel C:

Pyrenophora teres-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20° C und 100% relativer Luftfeuchtigkeit in einer Inkubationskabine.

Die Pflanzen werden in einem Gewächshaus bei einer Temperatur von ca. 20° C und einer relativen Luftfeuchtigkeit von ca. 80% aufgestellt.

7 Tage nach der Inokulation erfolgt die Auswertung.

Eine deutliche Überlegenheit in der Wirksamkeit gegenüber dem Stand der Technik zeigen bei diesem

Test z.B. die Verbindungen gemäß dem Herstellungsbeispiel 2.

Beispiel D:

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen 1, 2 und 4.

Beispiel E:

Pyricularia-Test (Reis)/protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% relativer Luftfeuchtigkeit und 25 °C aufgestellt.

Eine deutliche Überlegenheit in der Wirksamkeit gegenüber dem Stand der Technik zeigen in diesem Test z.B. die Verbindungen gemäß den Herstellungsbeispielen 1, 2, 3, 4 und 5.

## Ansprüche

**1.** N-Substituierte 3-Cyano-4-phenyl-pyrrole der allgemeinen Formel (I),

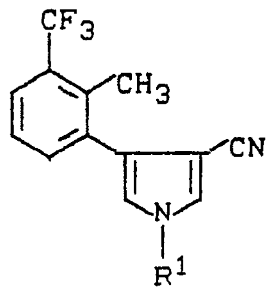

(I)

in welcher

$R^1$

für

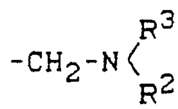 ; 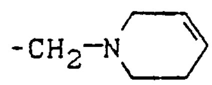 ; 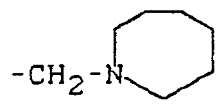 ;

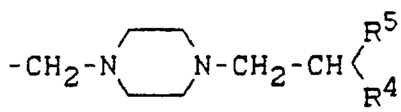 oder 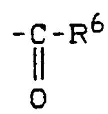 steht,

wobei

$R^2$

für gegebenenfalls substituiertes Alkyl, für Alkenyl, Alkinyl oder Cycloalkyl oder für jeweils gegebenenfalls substituiertes Aralkyl oder Aryl steht,

$R^3$

für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,

$R^4$

für Wasserstoff oder Alkyl steht,

$R^5$

für Cyano, Alkanoyl, Alkoxycarbonyl, Alkylaminocarbonyl, Dialkylaminocarbonyl, für Alkylsulfinyl, für Alkylsulfonyl, für Phenylsulfinyl oder für Phenylsulfonyl steht und

$R^6$

für Alkyl, Alkoxy, Alkoxyalkyl oder für Alkoxyalkyloxy steht.

**2.** N-Substituierte 3-Cyano-4-phenyl-pyrrole der Formel (I) gemäß Anspruch 1, bei welchen

$R^1$

für einen Rest

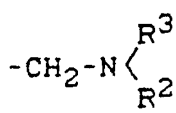 ; 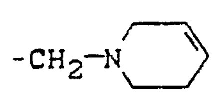 ; 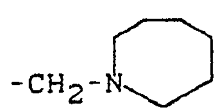 ;

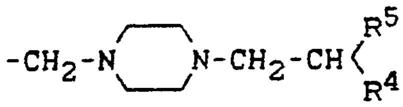 oder 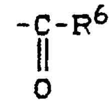 steht,

wobei

$R^2$

für gegebenenfalls substituiertes, geradkettiges oder verzweigtes Alkyl mit 1 bis 6 Kohlenstoffatomen steht, wobei als Substituenten infrage kommen: Cyano, Cycloalkyl mit 3 bis 7 Kohlenstoffatomen, jeweils geradkettiges oder verzweigtes Alkoxy, Alkylcarbonyl, Alkoxycarbonyl, Alkylaminocarbonyl, Dialkylaminocarbonyl, Alkylsulfinyl, Alkylsulfonyl oder Dialkylamino mit jeweils 1 bis 6 Kohlenstoffatomen in den einzelnen Alkylteilen; außerdem

$R^2$

für jeweils geradkettiges oder verzweigtes Alkenyl oder Alkinyl mit jeweils 3 bis 6 Kohlenstoffatomen, für Cycloalkyl mit 3 bis 7 Kohlenstoffatomen oder für jeweils gegebenenfalls einfach bis mehrfach, gleich oder verschieden im Phenylteil substituiertes Phenyl oder Benzyl steht, wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen,

$R^3$

für Heterocyclylalkyl mit 1 bis 6 Kohlenstoffatomen im Alkylteil, Heterocyclylalkenyl mit 3 bis 6 Kohlenstoffatomen im Alkenylteil, Heterocyclylalkinyl mit 3 bis 6 Kohlenstoffatomen im Alkinylteil oder Heterocyclyl steht, wobei Heterocyclyl jeweils für einen 5- bis 7-gliedrigen, gegebenenfalls einfach bis mehrfach, gleich oder verschieden substituierten, ungesättigen oder gesättigten Heterocyclus mit 1 bis 3 Heteroatomen, insbesondere Stickstoff, Sauerstoff oder Schwefel steht und wobei als Substituenten jeweils infrage kommen: Halogen, Cyano, jeweils geradkettiges oder verzweigtes Alkyl, Alkoxy, Halogenalkyl, Halogenalkoxy, Alkylcarbonyl oder Alkoxycarbonyl mit jeweils 1 bis 6 Kohlenstoffatomen in den einzelnen Alkylteilen und im Fall des Halogenalkyl- oder Halogenalkoxy-Restes mit jeweils 1 bis 9 gleichen oder verschiedenen Halogenatomen; außerdem $R^3$ für geradkettiges oder verzweigtes Dialkylaminoalkyl mit jeweils 1 bis 6 Kohlenstoffatomen in den einzelnen Alkylteilen steht, ferner für im Cycloalkylteil gegebenenfalls einfach bis mehrfach, gleich oder verschieden durch Halogen und geradkettigem oder verzweigtem Alkyl mit 1 bis 4 Kohlenstoffatomen substituiertes Cycloalkylalkyl mit 1 bis 6 Kohlenstoffatomen im geradkettigen oder verzweigten Alkylteil und 3 bis 7 Kohlenstoffatomen im Cycloalkylteil oder für im Phenylteil gegebenenfalls einfach bis mehrfach, gleich oder verschieden durch Cyano, Halogen, jeweils geradkettigem oder verzweigten Alkyl oder Alkoxy mit jeweils 1 bis 4 Kohlenstoffatomen substituiertes Phenylethyl steht,

$R^4$

für Wasserstoff oder für geradkettiges oder verzweigtes Alkyl mit 1 bis 6 Kohlenstoffatomen steht,

$R^5$

für Cyano oder für jeweils geradkettiges oder verzweigtes Alkanoyl, Alkoxycarbonyl, Alkylaminocarbonyl, Dialkylaminocarbonyl, Alkylsulfinyl oder Alkylsulfonyl mit jeweils 1 bis 6 Kohlenstoffatomen in den einzelnen Alkylteilen oder für Phenylsulfinyl oder Phenylsulfonyl steht und

$R^6$

für jeweils geradkettiges oder verzweigtes Alkyl, Alkoxy, Alkoxyalkyl oder Alkoxyalkyloxy mit jeweils 1 bis 6 Kohlenstoffatomen in den einzelnen Alkylteilen steht.

**3.** N-Substituierte 3-Cyano-4-phenyl-pyrrole der Formel (I) gemäß Anspruch 1,

bei welchem

$R^1$

für einen Rest

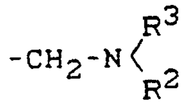 ; 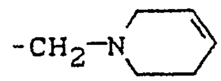 ; 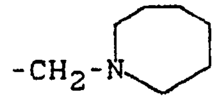 ;

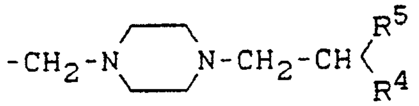 oder 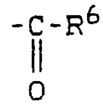 steht,

wobei

$R^2$

für jeweils gegebenenfalls substituiertes Methyl, Ethyl oder n- oder i-Propyl sowie n-, i- oder s-Butyl steht, wobei als Substituenten jeweils infrage kommen: Cyano, Methoxy, Ethoxy, Methylcarbonyl, Ethylcarbonyl, Methoxycarbonyl, Ethoxycarbonyl, Methylaminocarbonyl, Dimethylaminocarbonyl, Ethylaminocarbonyl, Diethylaminocarbonyl, Methylsulfinyl, Methylsulfonyl, Dimethylamino, Diethylamino oder Dipropylamino, Dibutylamino oder Cyclohexyl; außerdem $R^2$ für Allyl, n- oder i-Butenyl, Propargyl, n- oder i-Butinyl, für Cyclopropyl, Cyclopentyl oder Cyclohexyl, oder für jeweils gegebenenfalls ein- bis dreifach, gleich oder verschieden im Phenylteil substituiertes Benzyl oder Phenyl steht, wobei als Substituenten infrage kommen: Fluor, Chlor, Brom, Cyano, Methyl, Ethyl, n-oder i-Propyl, n-, i- oder s-Butyl, Methoxy, Ethoxy, Trifluormethyl, Trifluormethoxy, Acetyl, Methoxycarbonyl oder Ethoxycarbonyl,

$R^3$

für jeweils gegebenenfalls im Heterocyclylteil ein- oder zweifach, gleich oder verschieden substituiertes Heterocyclylmethyl, Heterocyclylethyl oder Heterocyclylpropyl steht, wobei als Heterocyclylrest jeweils einer der folgenden Reste infrage kommt

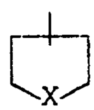 ; 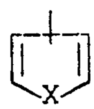 ; 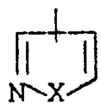 ; 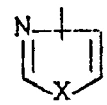 ; 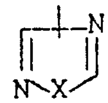 ;

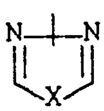 ; 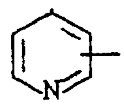 ; 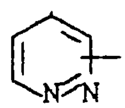 ; 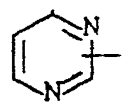 ; 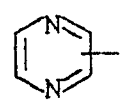 ;

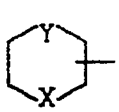 , 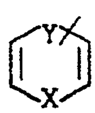 , oder 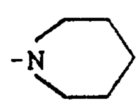

wobei X jeweils für Sauerstoff, Schwefel oder eine NH-Gruppe steht und Y jeweils für Sauerstoff, Schwefel, eine NH-Gruppe oder eine $CH_2$-Gruppe steht und wobei als Substituenten jeweils infrage kommen: Fluor, Chlor, Brom, Cyano, Methyl, Ethyl, n- oder i-Propyl, n-, i-, s- oder t-Butyl, Methoxy, Ethoxy, Trifluormethyl, Trifluormethoxy, Acetyl, Methoxycarbonyl oder Ethoxycarbonyl; außerdem $R^3$ für Dimethylaminoethyl, Diethylaminoethyl, Dimethylaminopropyl, Diethylaminopropyl, Dipropylaminopropyl, Dibutylaminopropyl, Dibutylaminobutyl, Dibutylaminoethyl, Dipropylaminoethyl, Dimethylaminobutyl, Diethylaminobutyl, Dimethylaminopentyl, Diethylaminopentyl, für jeweils gegebenenfalls ein- bis dreifach, gleich oder verschieden durch Methyl, Ethyl und Isopropyl substituiertes Cyclohexylmethyl

oder Cyclohexylethyl oder für gegebenenfalls ein- bis dreifach, gleich oder verschieden durch Fluor, Chlor, Brom, Cyano, Methyl, Ethyl, n-oder i-Propyl, n-, i-, s-oder t-Butyl, Methoxy, Ethoxy, n- oder i-Propoxy und n-, i-, s-oder t-Butoxy substituiertes Phenethyl steht,

$R^4$
für Wasserstoff, Methyl, Ethyl, n- oder i-Propyl sowie n-, i-, s- oder t-Butyl steht,

$R^5$
für Cyano, Acetyl, Propionyl, Methoxycarbonyl, Ethoxycarbonyl, Methylaminocarbonyl, Dimethylaminocarbonyl, Ethylaminocarbonyl, Diethylaminocarbonyl, Dipropylaminocarbonyl, Dibutylaminocarbonyl, Methylsulfinyl, Ethylsulfinyl, Methylsulfonyl, Ethylsulfonyl, Phenylsulfinyl oder Phenylsulfonyl steht und

$R^6$
für jeweils geradkettiges oder verzweigtes Alkyl, Alkoxy, Alkoxyalkyl oder Alkoxyalkyloxy

mit jeweils 1 bis 4 Kohlenstoffatomen in den einzelnen Alkylteilen steht.

**4.** N-Substituierte 3-Cyano-4-phenyl-pyrrole der Formel (I) gemäß Anspruch 1,

in welchen
$R^1$
für einen Rest

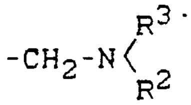 ; 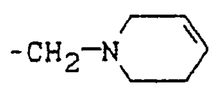 ; 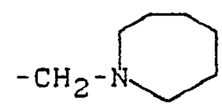 ;

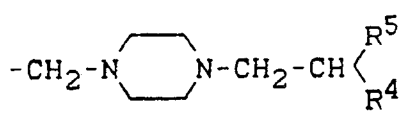 oder 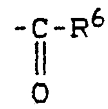 steht,

wobei

$R^2$
für Methyl, Ethyl, n- oder i-Propyl, n-, i-oder s-Butyl, Allyl, Propargyl, für Cyclohexyl, Cyclopentyl, Cyclopropyl, Cyclohexylmethyl, Phenyl, Benzyl oder für Cyanethyl steht,

$R^3$
für jeweils gegebenenfalls ein- bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

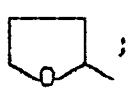 ; 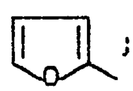 ; 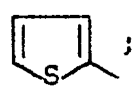 ; 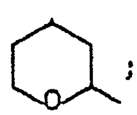 ; 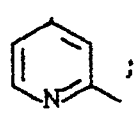 ;

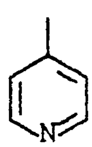 oder 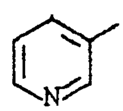 ;

oder für jeweils gegebenenfalls ein- bis dreifach, gleich oder verschieden durch Methyl und Ethyl

substituiertes 2-Heterocyclylethyl, 2-Heterocyclylpropyl oder 3-Heterocyclylpropyl steht, wobei als Heterocyclylreste infrage kommen:

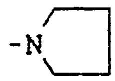 ; 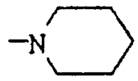 ; 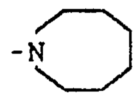 ; 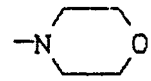 ;

außerdem für Diethylaminoethyl, Dimethylaminopropyl, Diethylaminopropyl, Dibutylaminopropyl oder Diethylaminopentyl, Cyclohexylmethyl oder Phenethyl steht, $R^4$ für Wasserstoff steht,

$R^5$
für Cyano, Acetyl, Propionyl, Methoxycarbonyl, Ethoxycarbonyl, Dimethylaminocarbonyl, Methylsulfinyl, Methylsulfonyl, Phenylsulfinyl oder Phenylsulfonyl steht und

$R^6$
für Methyl, Ethyl, Methoxy, Ethoxy, Methoxymethyl, Ethoxymethyl, Methoxymethyloxy, Methoxyethyloxy oder Ethoxyethyloxy steht.

5. N-Substituierte 3-Cyano-4-phenyl-pyrrole der Formel (I) gemäß Anspruch 1,

in welchen
$R^1$
für einen Rest

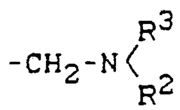 oder 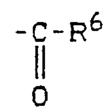 steht,

wobei

$R^2$
für Cyanethyl steht,

$R^3$
für jeweils gegebenenfalls ein- bis dreifach, gleich oder verschieden durch Chlor und Methyl substituiertes Heterocyclylmethyl, Heterocyclylethyl oder Heterocyclylpropyl steht, wobei als Heterocyclylreste infrage kommen:

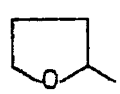 ; 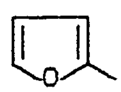 ; 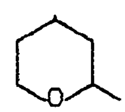 ; 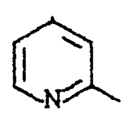 ;

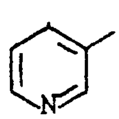 oder 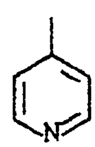 ;

oder für jeweils gegebenenfalls ein- bis dreifach, gleich oder verschieden durch Methyl und Ethyl substituiertes 2-Heterocyclylethyl, 2-Heterocyclylpropyl oder 3-Heterocyclylpropyl steht, wobei als Heterocyclylreste infrage kommen:

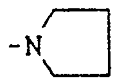 ; 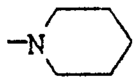 ; 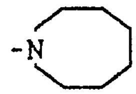 oder 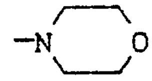 ;

außerdem für Diethylaminoethyl, Dimethylaminopropyl, Diethylaminopropyl, Dibutylaminopropyl, Diethylaminopentyl, Cyclohexylmethyl oder Phenethyl steht und

$R^6$
für Methyl, Ethyl, Methoxy, Ethoxy, Methoxymethyl, Ethoxymethyl, Methoxyethyloxy oder Ethoxyethyloxy steht.

**6.** N-Substituierte 3-Cyano-4-phenyl-pyrrole der Formel (I) gemäß Anspruch 1,

bei welchen
$R^1$
für einen Rest

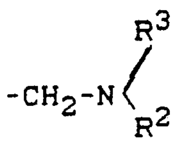 oder 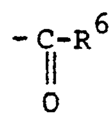 steht,

wobei

$R^2$
für Methyl, Ethyl, n- oder i-Propyl, n-, i-oder s-Butyl, Allyl, Propargyl, Cyclopropyl, Cyclopentyl, Cyclohexyl, Cyclohexylmethyl, Phenyl, Benzyl oder Cyanoethyl steht,

$R^3$
für gegebenenfalls ein- bis dreifach, gleich oder verschieden durch Chlor und Methyl substituiertes Heterocyclylmethyl steht, wobei als Heterocyclylreste infrage kommen:

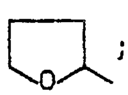 ; 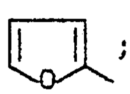 ; 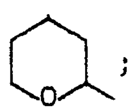 ; 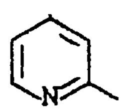 ; 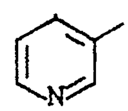

oder 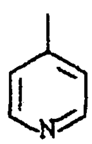 und

$R^6$
für Methyl, Ethyl, Methoxy, Ethoxy, Methoxymethyl, Ethoxymethyl, Methoxyethyloxy oder Ethoxyethyloxy steht.

**7.** Verfahren zur Herstellung von N-substituierten 3-Cyano-4-phenyl-pyrrolen dem allgemeinen Formel (I)

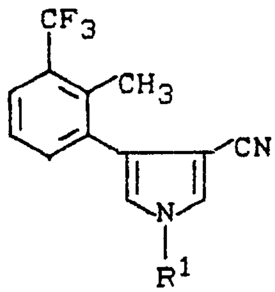

(I)

in welcher

$R^1$
für

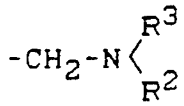 ; 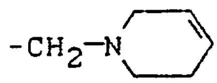 ; 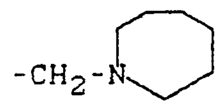 ;

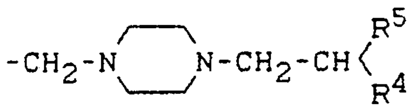 oder 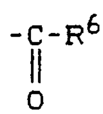 steht,

wobei

$R^2$
für gegebenenfalls substituiertes Alkyl, für Alkenyl, Alkinyl oder Cycloalkyl oder für jeweils gegebenenfalls substituiertes Aralkyl oder Aryl steht,

$R^3$
für jeweils gegebenenfalls substituiertes Heterocyclylalkyl, Heterocyclylalkenyl, Heterocyclylalkinyl oder Heterocyclyl, für Dialkylaminoalkyl oder für jeweils gegebenenfalls substituiertes Cycloalkylalkyl oder Phenethyl steht,

$R^4$
für Wasserstoff oder Alkyl steht,

$R^5$
für Cyano, Alkanoyl, Alkoxycarbonyl, Alkylaminocarbonyl, Dialkylaminocarbonyl, für Alkylsulfinyl, für Alkylsulfonyl, für Phenylsulfinyl oder für Phenylsulfonyl steht und

für Alkyl, Alkoxy, Alkoxyalkyl oder für $R^6$
Alkoxyalkyloxy steht,

dadurch gekennzeichnet, daß man
(a) das 3-(2-Methyl-3-trifluormethylphenyl)-4-cyanopyrrol der Formel (II)

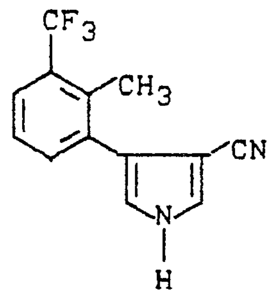

(II)

mit Formaldehyd und Aminen der Formel (III),

$$R^{1-1}\text{-H} \qquad (III)$$

in welcher

$R^{1-1}$
für einen Rest

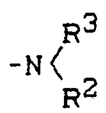 ; 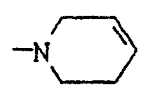 ; 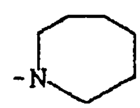

oder

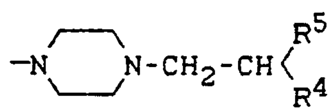 steht,

gegebenenfalls in Gegenwart eines Verdünnungsmittels und gegebenenfalls in Gegenwart eines Reaktionshilfsmittels umsetzt oder daß man,
(b) 3-(2-Methyl-3-trifluormethylphenyl)-4-cyano-pyrrol-Derivate der Formel (IV),

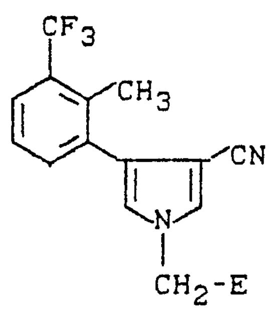

(IV)

in welcher

E
für eine elektronenanziehende Abgangsgruppe steht,

mit Aminen der Formel (III),

$$\text{H-}R^{1-1} \qquad (III)$$

in welcher

$R^{1-1}$
für einen Rest

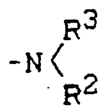 ; 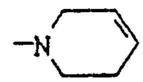 ; 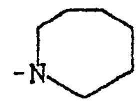

oder

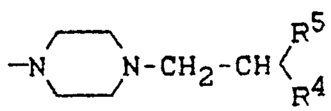 steht,

gegebenenfalls in Gegenwart eines Verdünnungsmittels und gegebenenfalls in Gegenwart eines Säurebindemittels umsetzt oder daß man
(c) das 3-(2-Methyl-3-trifluormethylphenyl)-4-cyano-pyrrol der Formel (II),

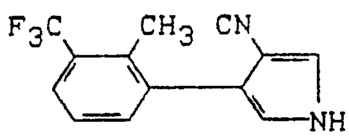

(II)

mit Acylierungsmitteln der Formel (V),

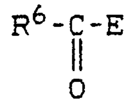

(V)

in welcher

$R^6$
die oben angegebene Bedeutung hat und

E
für eine elektronenanziehende Abgangsgruppe steht, gegebenenfalls in Gegenwart eines Verdünnungsmittels und gegebenenfalls in Gegenwart eines Reaktionshilfsmittels umsetzt.

8. Schädlingsbekämpfungsmittel, gekennzeichnet durch einen Gehalt an mindestens einem N-substituierten 3-Cyano-4-phenyl-pyrrol der Formel (I) nach den Ansprüchen 1 und 7.

9. Verwendung von N-substituierten 3-Cyano-4-phenyl-pyrrolen der Formel (I) nach den Ansprüchen 1 und 7 zur Bekämpfung von Schädlingen.

10. Verfahren zur Bekämpfung von Schädlingen, dadurch gekennzeichnet, daß man N-substituierte 3-Cyano-4-phenyl-pyrrole der Formel (I) nach den Ansprüchen 1 und 7 auf Schädlinge und/oder ihren Lebensraum einwirken läßt.

11. Verfahren zur Herstellung von Schädlingsbekämpfungsmitteln, dadurch gekennzeichnet, daß man N-substituierte 3-Cyano-4-phenyl-pyrrole der Formel (I) nach den Ansprüchen 1 und 7 mit Streckmitteln und/oder oberflächenaktiven Mitteln vermischt.

Europäisches Patentamt

# EUROPÄISCHER RECHERCHENBERICHT

Nummer der Anmeldung

EP 90123604.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | EP - A2/A3 - 0 133 247 (CIBA-GEIGY AG) * Patentansprüche 1,6,7,9, 11 * -- | 1,7-10 | C 07 D 207/34<br>C 07 D 401/12<br>C 07 D 405/12<br>C 07 D 409/12<br>A 01 N 43/36 |
| D,A | EP - A2/A3 - 0 281 731 (BAYER AG) * Patentansprüche 1,7-11 * -- | 1,7-11 | |
| D,A | EP - A2/A3 - 0 182 738 (CIBA-GEIGY AG) * Patentansprüche 1,23,24 * ---- | 1,8,10 | |

RECHERCHIERTE SACHGEBIETE (Int Cl 5)

C 07 D

Der vorliegende Recherchenbericht wurde für alle Patentansprüche erstellt.

| Recherchenort | Abschlußdatum der Recherche | Prüfer |
|---|---|---|
| WIEN | 01-02-1991 | HEIN |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82